# EUROPEAN PATENT SPECIFICATION

(11) **EP 1 187 650 B1**
(45) Date of publication and mention of the grant of the patent: **07.12.2005**
(21) Application number: 00936547.9
(22) Date of filing: 16.06.2000
(51) Int. Cl.: A61M 16/06

(54) **MASK AND HEADGEAR WITH CONNECTOR**
MASKE UND KOPFGURT MIT ANSCHLUSSTEIL
MASQUE ET CASQUE AVEC CONNECTEUR

(30) Priority: 18.06.1999 AU PQ104099; 18.06.1999 AU PQ191699; 16.12.1999 US 115618; 15.02.2000 US 504220
(43) Date of publication of application: 20.03.2002
(73) Proprietor: ResMed Ltd., North Ryde, NSW 2113 (AU)
(72) Inventor: GUNARATNAM, Michael, Kassipillai, Marsfield, NSW 2122 (AU); KWOK, Philip, Rodney, Chatswood, NSW 2113 (AU)
(74) Representative: Davies, Gregory Mark
(86) International application number: PCT/AU2000/000679
(87) International publication number: WO 2000/078383

(56) References cited:
- EP-A- 0 958 841
- WO-A-97/20597
- AU-A- 5 943 094
- GB-A- 684 788
- GB-A- 704 167
- US-A- 2 097 676
- US-A- 2 292 568
- US-A- 4 437 462

## Description

The invention relates to a mask and headgear suitable for the delivery of breathable gases to a patient for the treatment of sleep disordered breathing (SDB).

### Background of the Invention

Respiratory masks used in the treatment of SDB may comprise either a nasal mask, designed to fit over a patient's nose, or a full face mask designed to fit over the nose and mouth of the patient. Air or other breathable gas is supplied by a blower and passed along a flexible conduit to the mask.

The mask generally comprises a relatively rigid shell, termed a frame, which defines a rearwardly opening cavity covering the patient's nose and/or mouth and a soft portion, termed a cushion, which spaces the frame away from the face for comfortable contact.

The masks are typically held in place using headgear, the mask and headgear being joined using some form of connector.

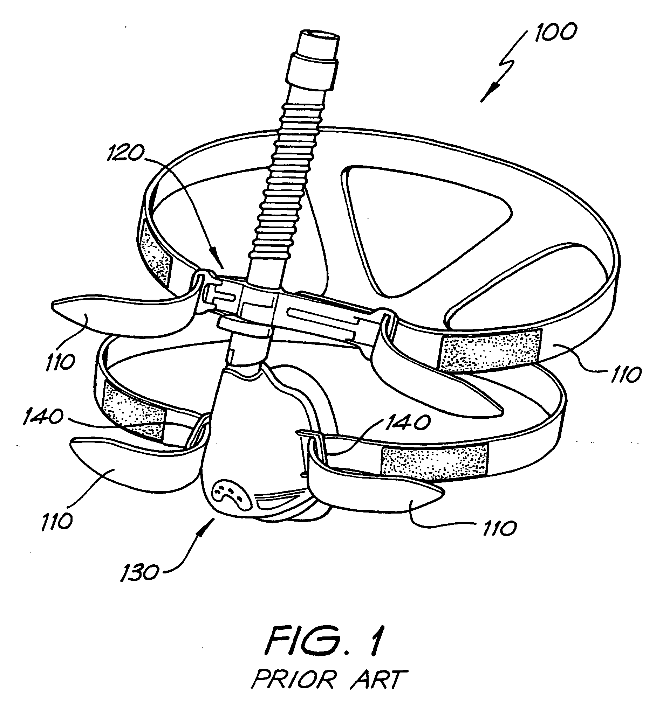

One known example is the Mirage® Mask (ResMed Limited), shown in Fig. 1. In this case, the headgear 100 is constructed from fabric and includes a rear portion, which engages the region near the occiput of the patient, and four straps 110 which are secured to a forehead support 120 (2 straps) and nasal mask frame 130 (2 straps). The straps include hook and loop material, such as Velcro(TM) on one side. The mask frame and forehead supports include loops 140 through which a strap can pass. In order to secure the mask in place on a patient's head, the four straps are passed through the four corresponding loops and held in place at an appropriate length by the hook and loop material. The patient can adjust the length of the material in order to secure a good fit of the mask. In some cases, it can require considerable adjustment to find the optimal length of straps which is required. A difficulty with this connector is that if the patient removes the mask by loosening the straps, they will lose what may have been a good fit of the mask the next time it is being used. In this case they will need to repeat the adjustment step.

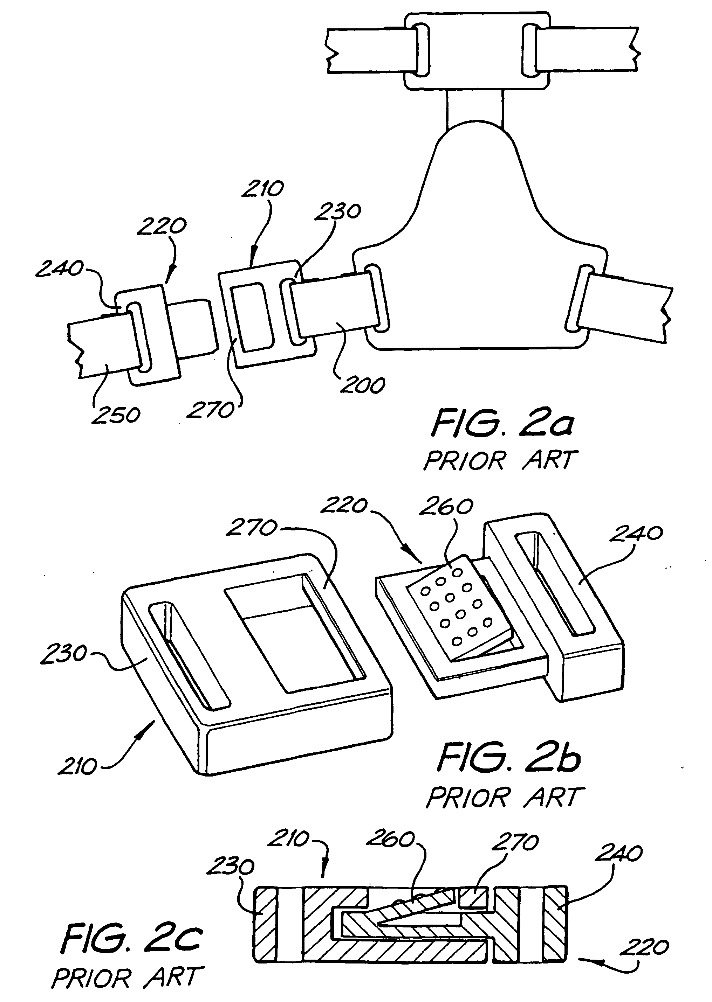

Another known manner of connecting the mask and headgear is shown in Figs. 2a, 2b and 2c. In this headgear, a short strap length 200 is secured to one side of the mask, with a two-part, press-release connector 210, 220 attaching this to the strap 250 of the headgear. One disadvantage of the approach of using this arrangement is that the connector may be difficult to release because the connector is free to move relative to the mask frame. A further disadvantage of this arrangement is that the connector may be in contact with the patient's face which may lead to discomfort in use, particularly if they sleep on their side.

More detailed views of this prior art connector are shown in Figs. 2b and 2c. Each part of the connector includes a bar 230, 240 behind which a respective one of the straps 200, 250 may be passed. The male portion 220 of the connector includes a resilient cantilever 260 which is captured behind a bar 270 on the female portion 210. The cantilever is depressed to engage and disengage the connector. A further disadvantage of this particular connector, best seen in Fig. 2b, is that it may be awkward to disengage the connector because of the close positioning of the bar 270 to the cantilever 260.

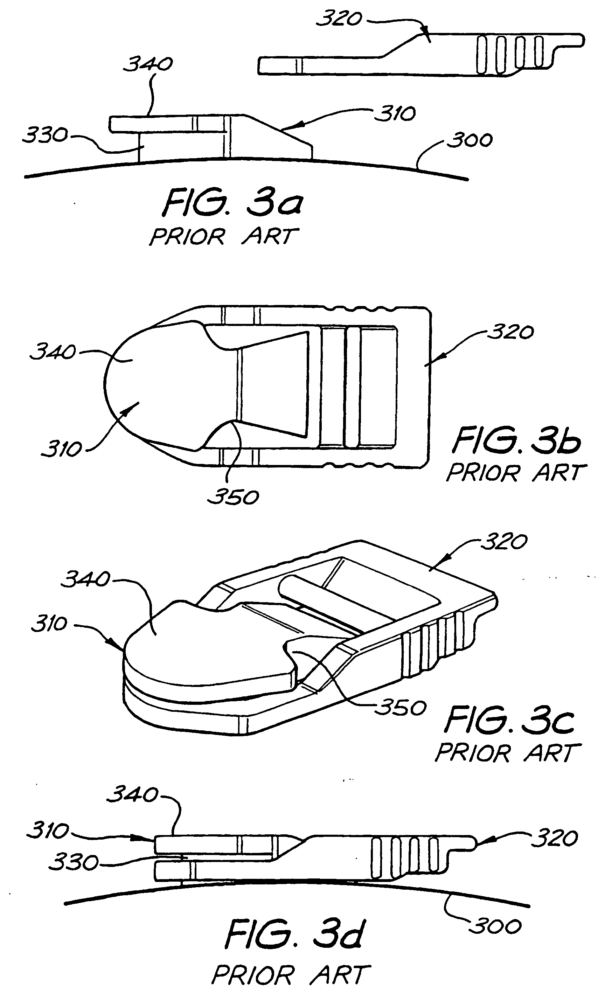

Another known mask and headgear connector is shown in Fig. 3a to Fig. 3d. This comprises a flexible part 310 positioned on the outer surface of a flexible mask frame 300 and a rigid part 320 formed generally as a D-ring with a loop to which the headgear strap is attached. The flexible part consists of a base 330 supporting an upper portion 340 (best illustrated in Fig. 3a) which overhangs the base portion 330 and has a narrow central region 350. In order to engage and disengage the connector, the flexible part 310 must be deformed whilst the D-ring of the rigid part is pushed over the upper portion 340 to engage below the overhanging ledge. This arrangement is awkward to engage and disengage and typically requires two hands.

Another known mask and headgear connector consists of hooks on the end of the headgear straps and corresponding holes in the mask frame. To engage the mask and headgear connector, the hook is passed through one of the corresponding holes on the headgear. This arrangement is also awkward to engage and disengage and typically requires two hands. Also, it is possible for the hooks to disengage during sleep as there is no locking means for the connection.

GB 704,167, showing the features of the preamble of claim 1, discloses an respiratory mask and headgear combination for administering anaesthesia, the mask comprising a rigid mask frame to which a pair of C-shaped clips are attached for connecting to respective ends of a headgear strap. Each C-shaped clip has an outwardly directed mouth, through which the strap can be inserted transverse itself once it has been compressed or folded along its longitudinal axis. An enlarged metal strip is fitted to each end of the strap, the strips being constrained by the clips against the tension of the strap to secure the mask against the patient's face.

There is a need for a connector arrangement which is simple and quick to operate.

### Summary of the Invention

In accordance with this invention, there is provided a respiratory mask and headgear combination comprising a respiratory mask having a rigid mask frame, adjustable headgear for securing said mask on a patient, said headgear including at least one attachment strap, whereby said mask frame has integrally and rigidly formed therewith a first connector portion, and a second connector portion adapted for releasable mating and locking with said first connector portion, said first and second connector portions being insertable, one into another, to form a press-release locked connection between said mask frame and said strap to allow single-handed engagement and disengagement by the patient in use, said second connector portion having means for connection of said attachment strap of the headgear.

### Brief Description of the Drawings

Embodiments of the invention will now be described with reference to the accompanying drawings, in which:
Fig. 1 shows the prior art Mirage® mask and headgear assembly.
Fig. 2a shows a front view of a prior art mask and headgear assembly.
Fig. 2b shows further detail of the prior art mask and headgear connector shown in Fig. 2a.
Fig. 2c shows a cross-sectional view of the prior art mask and headgear connector shown in Fig. 2b.
Fig. 3a shows a side view of another prior art mask and headgear connector where the two parts are not engaged.
Fig. 3b shows a top view of the prior art connector shown in Fig. 3a, where the two parts are engaged.
Fig. 3c shows a perspective view of the prior art connector shown in Fig. 3a, where the two parts are engaged.
Fig. 3d shows a side view of the prior art connector shown in Fig. 3a, where the two parts are engaged.
Fig. 4 shows a front view of a mask frame incorporating a female portion of the connector according to one embodiment of the invention.
Fig. 5 shows a perspective view of the mask frame of Fig. 4.
Fig. 6a shows a front view of a male portion of the connector adapted for use with the mask frame of Figs. 4 and 5.
Fig. 6b shows a side view of Fig. 6a.
Fig. 6c shows a sectional view along A-A of Fig. 6a.
Fig. 6d shows a perspective view of Fig. 6d.
Fig. 7a and 7b show an embodiment of the invention where the cross-bar on the male portions of the connectors have different angles.

### Detailed Description of the Invention

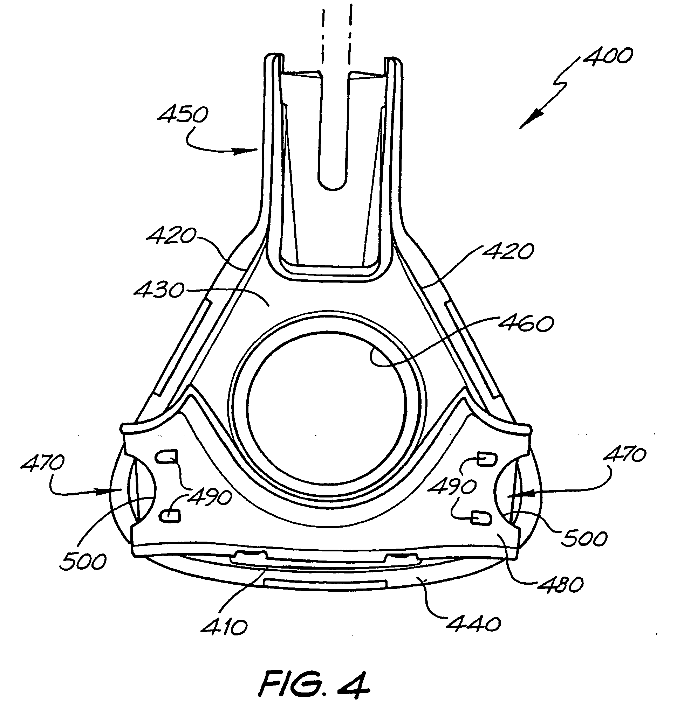

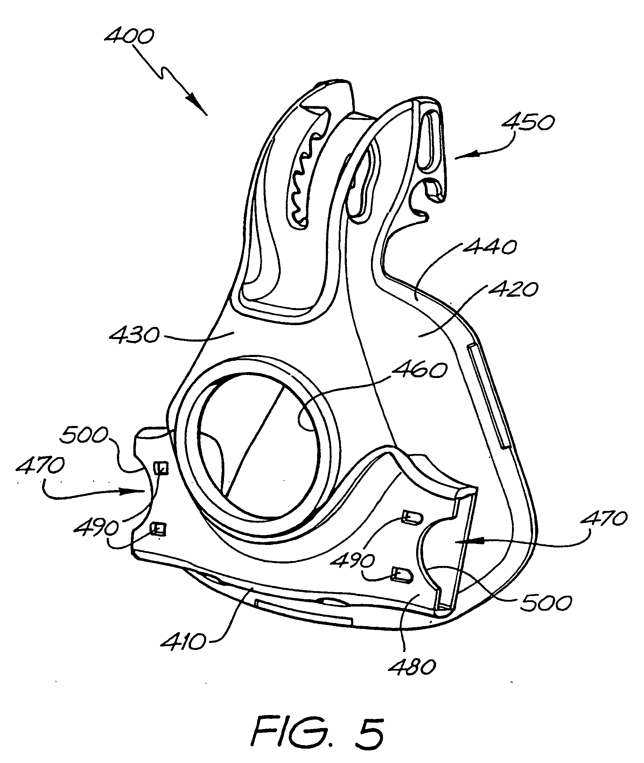

Figs. 4 and 5 illustrate a mask frame 400 for a nasal mask, formed as a moulded shell of polycarbonate or similar rigid material, which acts as a body onto which the other components of the mask are attached.

The frame 400 is generally triangular in front view, having a base 410, a pair of inclined side walls 420 extending towards an apex and a front wall 430. The frame defines a mask cavity covering the patient's nose, and is open at its rear. A rim 440 at the rear edge of the base 410 and side walls 420 approximates the contours of the patient's face and is adapted for attachment of a soft mask cushion (not shown) to space the frame away from the patient's face for sealing and comfort. The apex of the frame has an extension 450 for attachment of a forehead support (not shown).

In the illustrated mask frame, a gas inlet aperture 460 is formed in the front wall 430, for connection of a gas supply conduit or similar, which may include an elbow connector (not shown) pivotably connected to the frame. In other forms of mask, the gas inlet aperture may be formed at the apex of the frame.

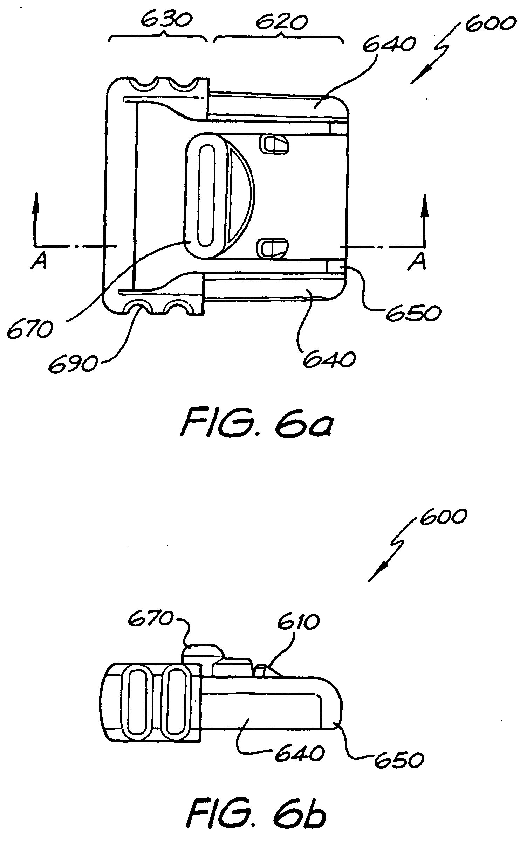

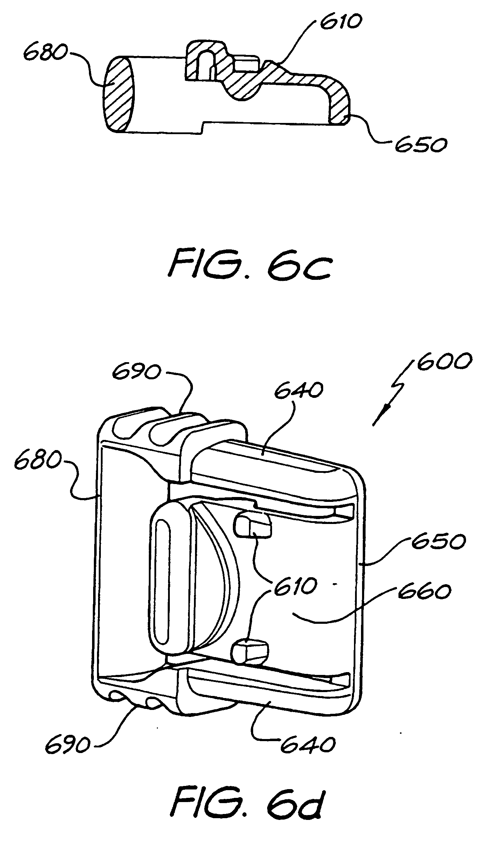

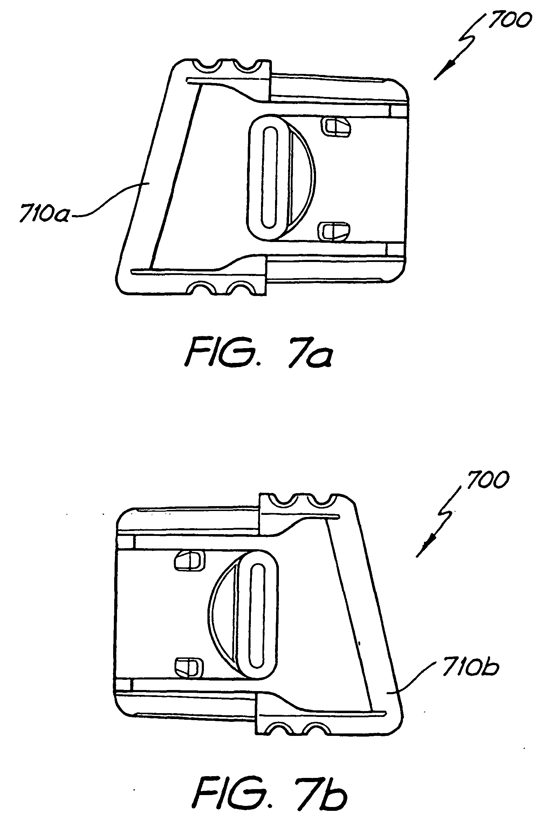

Integrally moulded into the lower corner regions of the rigid mask frame are a pair of female connector parts 470, for receiving the leading portions of the male connector parts 600, 700 illustrated in Figs. 6a to 6d, or Figs. 7a and 7b. These female connector parts open generally to the side of the mask, but are angled slightly downwards and rearwards (relative to the orientation of the mask frame) so that they hold the male connectors approximately parallel to that part of the patient's cheek region which the connectors overlie. The connectors are held clear of the patient's face by the rigid mask frame 400.

The female connectors 470 each define a recess which opens towards a side of the mask and which approximates a rounded-cornered rectangular in end view, adapted to provide a close fit with the corresponding male connectors 600,700 when engaged. These end shapes are preferably slightly asymmetric (keyed) so as to prevent upside-down insertion of the male connector.

A front wall 480 of the female connector has a pair of sockets 490 at least on its inner surface, for receiving respective lugs 610 of the male connector 600. The end of the front wall may also have a curved cut-out portion 500, as will be described below.

A corresponding male connector part 600 is illustrated in Figs. 6a to 6d.

The male connector has a leading portion 620 which is received in the recess of the female connector 470, and a trailing portion 630 which remains outside the recess. The leading portion includes upper and lower side beams 640 connected by a cross-piece 650 at their leading ends, and a resiliently biased cantilever member 660 depending from the cross-piece 650 and extending back towards the trailing portion. The cantilever has on its front surface the lugs 610, and a ridge 670 at its trailing edge.

The locating lugs 610 have a rounded wedge profile so as to allow reduce wear and provide smooth engagement. The wedge profile does allow insertion of the male connector 600 into the female connector 470 on the mask without depression of the cantilever, although in preferred operation the patient will depress the cantilever by pushing on ridge 670 for both engagement and disengagement. Once the male connector has been inserted sufficiently to snap the locating lugs 610 into engagement with corresponding sockets 490 of the female connector, disengagement can occur only by depression of the cantilever.

The trailing portion 630 of the male connector has a cross-bar 680 forming a loop through which the headgear strap can be passed and adjusted for proper fit. Upper and lower sides 690 of the trailing portion have grooves or other formations to make it more easily gripped by the patient's fingers, while the distance between the leading side of the cross-bar 680 and the trailing edge and ridge 670 of the cantilever is preferably at least 5mm to allow easy connection of the strap and provide sufficient space for a finger to push on the cantilever ridge, even when the strap is attached. The arcuate cut-out 500 in the female connector front wall allows clearance for an end of the finger to overhang the ridge 670 when the ridge is being contacted by the pad of the finger.

Figs 7a and 7b illustrate a male connector 700 which is a variation on that of Figs 6a to 6d, in that the cross bars 710a, 710b for attachment of the headgear strap are set at an angle.

The shape, dimensions and position of the connectors may be optimised for most convenient use. For example, the male connector may be approximately 20 mm wide, 25 mm long and 10 mm thick. The cantilever 660 is approximately 15 mm long. The locating lugs 610 are approximately 3 mm long, 2 mm wide and 1 mm high, and the ridge is approximately 14 mm wide and has a length of approximately 5 mm.

The female connectors 470 are oriented on the mask frame so that in use the straps of the headgear are aligned to be approximately parallel with the sides of the face with which they make contact. When viewed from the front of the mask (the view in Fig. 4), the tops of the left and right hand side male connector's cross-bars 680 are further apart than the bottoms of the cross-bars. When viewed from the top the mask, the connectors are aligned to be positioned on an arc of an ellipse. An advantage from this arrangement is that when straps are connected and tensioned, the line of force will be more evenly transferred from the mask to the frame.

Both portions of the connector are preferably constructed from polycarbonate, such as Makrolon 2458 polycarbonate from Bayer, or similar rigid plastics material. The female portions of the connectors are clear and desirably are integrally moulded with the mask frame which is formed of the same polycarbonate material, thus ensuring a permanent, rigid attachment of the female connector to the mask frame and minimising the number of separate parts. The male portions may be frosted or textured. An advantage from using a frosted or slightly textured surface is that the male portion may be easier to distinguish from the female portion by touch. This is an advantage in the dark, the typical time when the mask is being used by a patient.

The configuration thus allows single-hand operation of the connector using, for example, thumb and index finger to grip the grooved sides 690 of the male connector and the middle finger to depress the cantilever. The connectors may be located on both the left and right hand side of the mask. In this way persons may use which ever side is most convenient to them. Alternatively the frame may be formed with only one connector, to reduce manufacturing costs.

In one unillustrated embodiment, the press release mechanism may be adapted to be operated by pressing the top and/or bottom sides of the male connector.

By rigidly forming one part of the connector onto the mask frame, the patient does not need to use both hands to disconnect the mask for removal. Thus, the arrangement facilitates quick and easy disconnection even though the connector will be out of patient's line of vision and the patient may be less than fully awake or in an anxious state.

A further advantage of using female portions 470 integrated with the mask frame is that they result in a reduced dead volume of the mask frame.

While particular embodiments of this invention have been described, it will be evident to those skilled in the art that the present invention may be embodied in other specific forms without departing from the essential characteristics thereof. The present embodiments and examples are therefore to be considered in all respects as illustrative and not restrictive, the scope of the invention being indicated by the appended claims rather than the foregoing description.

## Claims

1. A respiratory mask and headgear combination comprising a respiratory mask having a rigid mask frame (400), adjustable headgear for securing said mask on a patient, said headgear including at least one attachment strap, whereby said mask frame has a first connector portion (470), and a second connector portion (600) adapted for releasable mating with said first connector portion (470), said second connector portion (600) having means for connection of said attachment strap of the headgear **characterised in that** said first connector portion (470) is integrally end rigidly formed with the next frame (400) and said first and second connector portions (470, 600) are insertable, one into another, to form a press-release locked connection between said mask frame (400) and said strap to allow single-handed engagement and disengagement by the patient in use.

2. A respiratory mask and headgear combination according to claim 1 **characterised in that** said first connector (470) is a female connector portion.

3. A respiratory mask and headgear combination according to claim 2 **characterised in that** said second connector portion is a male connector portion (600).

4. A respiratory mask and headgear combination according to claim 3 **characterised in that** said male connector portion (600) includes a resiliently biassed cantilever member (660) depending from a leading end portion of said male connector portion and having means (610) for locking engagement with the female connector portion (470).

5. A respiratory mask and headgear combination according to claim 4 **characterised in that** said cantilever member (660) has a leading end, a trailing end, locking means (610) located intermediate said leading end and trailing end for engaging with said first connector portion (470), and release means (670) located adjacent said trailing end.

6. A respiratory mask and headgear combination according to claim 5 **characterised in that** said release means (670) comprises a raised portion adjacent a trailing end of said cantilever member.

7. A respiratory mask and headgear combination according to claim 6 **characterised in that** a space is provided immediately behind said trailing end of the cantilever member.

8. A respiratory mask and headgear combination according to claim 7 **characterised in that** said locking means comprises at least one lug (610) on a forward surface of said cantilever member, said lug engaging a corresponding socket (490) of said first connector portion (470).

9. A respiratory mask and headgear combination according to any one of claims 1-8 **characterised in that** said first and second connector portions are spaced forwardly of the patient's face by said rigid mask frame.

10. A respiratory mask and headgear combination according to any one of claims 1-3 and 9, **characterised in that** said second connector portion (600) has first and second gripping surfaces (690) positioned for gripping of said second connector portion (600) between a thumb and finger of a patient's hand, and release means (670) positioned for operation by another finger of the patient's hand.

11. A respiratory mask and headgear combination according to claim 10 **characterised in that** said release means (670) is pressed by said finger to depress a resilient cantilever member (660) of said second connector (600).

12. A respiratory mask and headgear combination according to claim 11 **characterised in that** said release means (670) comprises a raised portion adjacent a trailing end of said cantilever member (660).

13. A respiratory mask and headgear combination according to claim 12 **characterised in that** a space is provided immediately behind said trailing end of the cantilever member (660).

14. A respiratory mask and headgear combination according to claim 13 **characterised in that** said cantilever member (660) includes locking means (610) located intermediate said raised portion and a leading end of said cantilever member (660).

15. A respiratory mask and headgear combination according to claim 14 **characterised in that** said locking means comprises at least one lug (610) on a forward surface of said cantilever member (660), said lug (610) engaging a corresponding socket (490) of said first connector portion (470).

16. A respiratory mask and headgear combination according to claim 15 **characterised in that** a leading surface of said lug (610) is ramped.

17. A respiratory mask and headgear combination according to any one of claims 10-16 **characterised in that** said second connector portion (600) is adapted, in use, for gripping of said first and second gripping surfaces by the thumb and index finger of the patient's hand.

## Patentansprüche

1. Beatmungsmasken- und Kopfhalterungskombination mit einer Beatmungsmaske, die einen steifen Maskenrahmen (400) aufweist, einer einstellbaren Kopfhalterung zum Sichern der Maske an einem Patienten, wobei die Kopfhalterung mindestens einen Befestigungsgurt umfasst, wobei der Maskenrahmen ein erstes Verbindungsteil (470) und ein zweites Verbindungsteil (600) aufweist, welches zum lösbaren Verbinden mit dem ersten Verbindungsteil (470) geeignet ist, und wobei das zweite Verbindungsteil (600) Mittel zur Verbindung des Befestigungsgurtes der Kopfhalterung aufweist,
**dadurch gekennzeichnet, dass** das erste Verbindungsteil (470) integriert in und starr mit dem Ende des Maskenrahmens (400) gebildet ist, dass die ersten und zweiten Verbindungsteile (470, 600) ineinander einführbar sind, um eine drucklösende Verschlussverbindung zwischen dem Maskenrahmen (400) und dem Gurt zu bilden, um eine einhändige Ankopplung und Abkopplung durch den Patienten bei Verwendung zu ermöglichen.

2. Beatmungsmasken- und Kopfhalterungskombination nach Anspruch 1,
**dadurch gekennzeichnet, dass** das erste Verbindungsteil (470) ein Gurtschloss ist.

3. Beatmungsmasken- und Kopfhalterungskombination nach Anspruch 2,
**dadurch gekennzeichnet, dass** das zweite Verbindungsteil ein Gurtstecker (600) ist.

4. Beatmungsmasken- und Kopfhalterungskombination nach Anspruch 3,
**dadurch gekennzeichnet, dass** der Gurtstecker (600) einen elastisch vorgespannten freitragenden Teil (660), der mit einem vorderen Endteil des Gurtsteckers gekoppelt ist und Mittel zum schließenden Ankoppeln an das Gurtschloss (470) aufweist.

5. Beatmungsmasken- und Kopfhalterungskombination nach Anspruch 4,
**dadurch gekennzeichnet, dass** der freitragende Teil (660) ein vorderes Ende, ein hinteres Ende, ein Schließmittel (610), das zwischen dem vorderen Ende und dem hinterem Ende zum Ankoppeln an das erste Verbindungsteil (470) angeordnet ist, und ein Lösmittel (670), das benachbart zu dem hinteren Ende angeordnet ist, aufweist.

6. Beatmungsmasken- und Kopfhalterungskombination nach Anspruch 5,
**dadurch gekennzeichnet, dass** das Lösmittel (670) ein erhöhtes Teil benachbart zu einem hinteren Ende des freitragenden Teils umfasst.

7. Beatmungsmasken- und Kopfhalterungskombination nach Anspruch 6,
**dadurch gekennzeichnet, dass** unmittelbar hinter dem hinteren Ende des freitragenden Teils eine Aussparung vorgesehen ist.

8. Beatmungsmasken- und Kopfhalterungskombination nach Anspruch 7,
**dadurch gekennzeichnet, dass** das Schließmittel mindestens einen Vorsprung (610) auf einer vorderen Oberfläche des freitragenden Teiles aufweist, wobei der Vorsprung an eine entsprechende Ausnehmung (490) des ersten Verbindungsteils (470) ankoppelt.

9. Beatmungsmasken- und Kopfhalterungskombination nach einem der Ansprüche 1 bis 8.
**dadurch gekennzeichnet, dass** die ersten und zweiten Verbindungsteile durch den steifen Maskenrahmen vor dem Gesicht des Patienten beabstandet sind.

10. Beatmungsmasken- und Kopfhalterungskombination nach einem der Ansprüche 1 bis 3 und 9.
**dadurch gekennzeichnet, dass** der zweite Verbindungsteil (600) erste und zweite Griffoberflächen (690), die zum Anfassen des zweiten Verbindungsteiles (600) zwischen einem Daumen und Finger einer Hand des Patienten angeordnet sind, und ein Lösmittel (670), das zur Betätigung durch einen weiteren Finger der Hand des Patienten angeordnet ist, aufweist.

11. Beatmungsmasken- und Kopfhalterungskombination nach Anspruch 10,
**dadurch gekennzeichnet, dass** das Lösmittel (670) durch den Finger gedrückt wird, um einen elastischen freitragenden Teil (660) des zweiten Verbindungsteiles (600) herabzudrücken.

12. Beatmungsmasken- und Kopfhalterungskombination nach Anspruch 11,
**dadurch gekennzeichnet, dass** das Lösmittel (670) ein erhöhtes Teil benachbart zu einem hinteren Ende des freitragenden Teiles (660) aufweist.

13. Beatmungsmasken- und Kopfhalterungskombination nach Anspruch 12,
**dadurch gekennzeichnet, dass** unmittelbar hinter dem hinteren Ende des freitragenden Teiles (660) eine Aussparung vorhanden ist.

14. Beatmungsmasken- und Kopfhalterungskombination nach Anspruch 13,
**dadurch gekennzeichnet, dass** der freitragende Teil (660) ein Schließmittel (610) aufweist, das zwischen dem erhöhten Teil und einem vorderen Ende des freitragenden Teiles (660) angeordnet ist.

15. Beatmungsmasken- und Kopfhalterungskombination nach Anspruch 14,
**dadurch gekennzeichnet, dass** das Schließmittel mindestens einem Vorsprung (610) auf einer vorderen Oberfläche des freitragenden Teiles (660) aufweist, wobei der Vorsprung (610) an eine entsprechenden Ausnehmung (490) des ersten Verbindungsteiles (470) ankoppelt.

16. Beatmungsmasken- und Kopfhalterungskombination nach Anspruch 15,
**dadurch gekennzeichnet, dass** eine vordere Oberfläche des Vorsprungs (610) als Rampe geformt ist.

17. Beatmungsmasken- und Kopfhalterungskombination nach einem der Ansprüche 10 bis 16,
**dadurch gekennzeichnet, dass** das zweite Verbindungsteil (600) bei Verwendung zum Anfassen der ersten und zweiten Griffoberflächen durch den Daumen und Zeigefinger der Hand des Patienten geeignet ist.

## Revendications

1. Combinaison d'un masque respiratoire et d'un casque comprenant un masque respiratoire ayant un cadre de masque rigide (400), un casque réglable pour fixer ledit masque sur un patient, ledit casque comportant au moins une bande d'attache, dans laquelle ledit cadre de masque a une première partie (470) de connecteur, et une seconde partie (600) de connecteur propre à coopérer de façon amovible avec ladite première partie (470) de connecteurs, ladite seconde partie (600) de connecteur ayant des moyens pour la connexion de ladite bande d'attache du casque, **caractérisée en ce que** ladite première partie (470) de connecteur est formée d'une seule pièce et de façon rigide avec l'extrémité du cadre (400) de masque, lesdites première et seconde parties (470,600) sont insérables l'une dans l'autre pour former une connexion verrouillée qui se détache par pression, entre ledit cadre (400) de masque et ladite bande pour permettre l'engagement et le dégagement d'une seule main par le patient qui l'utilise.

2. Combinaison d'un masque respiratoire et d'un casque selon la revendication 1, **caractérisée en ce que** ledit premier connecteur (470) est une partie de connecteur femelle.

3. Combinaison d'un masque respiratoire et d'un casque selon la revendication 2, **caractérisée en ce que** ladite seconde partie de connecteur est une partie (600) de connecteur mâle.

4. Combinaison d'un masque respiratoire et d'un casque selon la revendication 3, **caractérisée en ce que** ladite partie (600) de connecteur mâle comporte un élément (660) en porte-à-faux, forcé de façon élastique , solidaire d'une partie frontale d'extrémité de ladite partie (600)de connecteur mâle, et ayant des moyens pour un engagement à verrouillage avec la partie (470) de connecteur femelle.

5. Combinaison d'un masque respiratoire et d'un casque selon la revendication 4, **caractérisée en ce que** ledit élément (660) en porte-à-faux a une extrémité frontale, une extrémité arrière, des moyens (610) de verrouillage disposés entre ladite extrémité frontale et ladite extrémité arrière pour s'engager avec ladite première partie (470) de connecteur et des moyens (670) de déverrouillage disposés au voisinage de ladite extrémité arrière.

6. Combinaison d'un masque respiratoire et d'un casque selon la revendication 5, **caractérisée en ce que** lesdits moyens (670) de déverrouillage comprennent une partie surélevée au voisinage d'une extrémité arrière dudit élément en porte-à-faux.

7. Combinaison d'un masque respiratoire et d'un casque selon la revendication 6 **caractérisée en ce qu'**un espace est prévu immédiatement derrière ladite extrémité arrière dudit élément en porte-à-faux.

8. Combinaison d'un masque respiratoire et d'un casque selon la revendication 7, **caractérisée en ce que** lesdits moyens de verrouillage comprennent au moins une patte (610) sur une surface avant dudit élément en porte-à-faux, lesdites pattes s'engageant dans une douille correspondante (490) de ladite première partie (470) de connecteur.

9. Combinaison d'un masque respiratoire et d'un casque selon l'une quelconque des revendications 1 à 8, **caractérisée en ce que** lesdites première et seconde parties de connecteurs sont espacés vers l'avant du visage du patient par ledit cadre de masque rigide.

10. Combinaison d'un masque respiratoire et d'un casque selon l'une des revendications 1 à 3 et 9, **caractérisée en ce que** ladite seconde partie (600) de connecteur a des première et seconde surfaces (640) de préhension, positionnées pour la préhension de ladite seconde partie (600) de connecteur entre un pouce et un doigt de la main du patient, et des moyens (670) de déverrouillage positionnés pour fonctionner avec un autre doigt de la main du patient.

11. Combinaison d'un masque respiratoire et d'un casque selon la revendication 10, **caractérisée en ce que** lesdits moyens (670) de déverrouillage sont pressés par ledit doigt pour soulager un élément (660) en porte-à-faux élastique dudit second connecteur (600) .

12. Combinaison d'un masque respiratoire et d'un casque selon la revendication 11 **caractérisée en ce que** lesdits moyens (670) de déverrouillage comprennent une partie surélevée au voisinage d'une extrémité avant dudit élément (660) en porte-à-faux.

13. Combinaison d'un masque respiratoire et d'un casque selon la revendication 12, **caractérisée en ce qu'**un espace est prévu immédiatement derrière ladite extrémité arrière dudit élément (660) en porte-à-faux.

14. Combinaison d'un masque respiratoire et d'un casque selon la revendication 13, **caractérisée en ce que** ledit élément (660) en pvrte-à-faux comporte des moyens de verrouillage (610) disposés entre ladite partie surélevée et une extrémité frontale dudit élément (660) en porte-à-faux.

15. Combinaison d'un masque respiratoire et d'un casque selon la revendication 14 **caractérisée en ce que** lesdits moyens de verrouillage comprennent au moins une patte (610) sur une surface avant dudit élément (660) en porte-à-faux, ladites patte (610) coopérant avec une douille (490) correspondante de ladite première partie (470) de connecteur.

16. Combinaison d'un masque respiratoire et d'un casque selon la revendication 15 **caractérisée en ce qu'**une surface frontale de ladite patte (610) forme une rampe.

17. Combinaison d'un masque respiratoire et d'un casque selon l'une quelconque des revendications 10 à 16, **caractérisée en ce que** ladite seconde partie (600) de connecteur est adaptée pour, en cours d'utilisation, la préhension desdites première et seconde surface par le pouce et l'index de la main du patient.
